(19) Europäisches Patentamt
European Patent Office
Office européen des brevets

(11) Veröffentlichungsnummer: **0 199 209 B1**

# (12) EUROPÄISCHE PATENTSCHRIFT

(45) Veröffentlichungstag der Patentschrift: **26.10.88**

(51) Int. Cl.⁴: **C 07 C 15/02,** C 07 C 5/367, C 07 C 5/387

(21) Anmeldenummer: **86104967.4**

(22) Anmeldetag: **11.04.86**

(54) **Verfahren zur Herstellung von Alkylbenzolen.**

(30) Priorität: **16.04.85 DE 3513569**

(43) Veröffentlichungstag der Anmeldung: **29.10.86 Patentblatt 86/44**

(45) Bekanntmachung des Hinweises auf die Patenterteilung: **26.10.88 Patentblatt 88/43**

(84) Benannte Vertragsstaaten: **BE CH DE FR GB IT LI NL**

(56) Entgegenhaltungen:
**EP - A - 0 022 297**
**DE - B - 1 268 124**
**US - A - 4 300 010**
**US - A - 4 429 175**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Fischer, Rolf, Dr., Bergstrasse 98, D-6900 Heidelberg (DE)**
Erfinder: **Mross, Wolf Dieter, Dr., Anselm-Feuerbach-Strasse 21, D-6710 Frankenthal (DE)**
Erfinder: **Pape, Frank-Friedrich, Dr., Berner Weg 34, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Anmeldung betrifft ein Verfahren zur Herstellung von Alkylbenzolen durch Umsetzung von Alkyl-, Alkyliden- oder Alkenylcyclohexenen sowie Alkyl- oder Alkenylcyclohexadienen an Katalysatoren.

Es ist bekannt, Vinylcyclohexen an Edelmetallkatalysatoren wie Palladium, Platin, Ruthenium oder Iridium auf basischen Trägern (EP-PS 22 297) in Ethylbenzol zu verwandeln. Es ist weiterhin bekannt, Limonen an Nickel- und Molybdänoxid enthaltenen Trägerkatalysatoren bei Temperaturen zwischen 200 und 350°C zu Gemischen aus p-Cymol, trans-p-Menthan und 3-p-Menthen umzusetzen (US-Patent 3 312 635). Anstelle von Nickel- und Molybdänoxid-Katalysatoren sind in Gegenwart von Wasserstoff auch Magnesiumoxid, Calciumoxid oder Lanthanoxid geeignet (Bull. Chem. Soc. Jpn. 1978, Band 51, Seiten 3641 bis 3642). p-Cymol ist weiterhin durch Umsetzung von Limonen, α-Pinen, β-Pinen oder Limonen enthaltendem Terpentinöl an Alkalimetallcarbonaten auf Trägern zugänglich (EP-PS 77 289).

In allen genannten Fällen werden Katalysatoren verwendet, bei denen entweder der Katalysatorträger oder die auf dem Katalysatorträger befindlichen aktiven Komponenten basische Stoffe wie Metalloxide oder Metallcarbonate darstellen.

Das US-Patent 2 976 331 beschreibt die Umsetzung von cyclischen Olefinen an Metall-Aluminiumsilikaten mit Porendurchmessern von 10 bis $13 \cdot 10^{-10}$ m zu Aromaten. Die Selektivität dieser grossporigen Zeolithe ist ausgesprochen schlecht.

In den US-Patenten 4 300 010 und 4 429 175 wird gelehrt, dass sich Vinylcyclohexen zu Ethylbenzol dehydrieren lässt, wenn als Katalysator ein nicht-acider, Palladium-dotierter Aluminiumsilikatzeolith mit einem Porendurchmesser von weniger als $5 \cdot 10^{-10}$ m, z.B. Zeolith A, verwendet wird und man die Umsetzung in Gegenwart von molekularem Sauerstoff durchführt. Der Zeolith dient hierbei als Träger für die Aktivkomponente Palladium. Voraussetzung für gute Aktivität und Selektivität ist eine aufwendige schrittweise Calcinierung des Katalysators, wobei zunächst in Gegenwart von molekularem Sauerstoff und dann in einer Wasserstoffatmosphäre oder einer $C_1$- bis $C_{10}$-nichtaromatische Kohlenwasserstoffe enthaltenden Atmosphäre calciniert wird.

Der Erfindung lag nun die Aufgabe zugrunde, einen Katalysator zur Verfügung zu stellen, der es ermöglicht, verschiedenartig substituierte Cyclohexene oder Cyclohexadiene in Alkylbenzole zu überführen. Der Katalysator sollte sich durch einfache Verfügbarkeit, lange Standzeiten, hohe Aktivität und leichte Regenerierbarkeit auszeichnen und hohe Umsätze mit guter Selektivität gewährleisten.

Es wurde nun gefunden, dass sich Alkylbenzole durch Umsetzung von Alkyl-, Alkyliden- und/oder Alkenylcyclohexenen sowie Alkyl- oder Alkenylcyclohexadienen Gegenwart eines Katalysators vorteilhaft in der Weise herstellen lassen, dass man acide zeolithische Katalysatoren des Pentasiltyps in der Gas- oder Flüssigphase verwendet.

Im erfindungsgemässen Verfahren werden die eingangs an den Katalysator gestellten Anforderungen weitgehend erfüllt. Im Hinblick auf den Stand der Technik, der die Umsetzung an nicht aciden Katalysatoren lehrt, ist das Gelingen des Verfahrens besonders überraschend. Anstelle von engporigen Aluminiumsilikatzeolithen werden mittelporige Zeolithe des Pentasiltyps verwendet. Die Dehydrierung kann in Abwesenheit von Sauerstoff durchgeführt werden, wodurch ein wesentlicher Beitrag zur Sicherheit der technischen Durchführung geleistet wird.

Die Reaktion lässt sich bei der Verwendung von Vinylcyclohexen als Ausgangsstoff für die Herstellung von Ethylbenzol durch folgende Formeln wiedergeben:

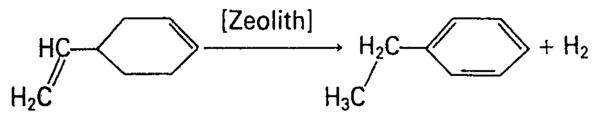


Für die erfindungsgemässe Umsetzung kommen als Ausgangsstoffe z.B. alkylsubstituierte Cyclohexene oder Cyclohexadiene in Betracht, wobei der Alkylsubstituent 1 bis 10, vorzugsweise 1 bis 5 Kohlenstoffatome enthält und geradkettig oder verzweigt ist. Beispielsweise kann als Alkylgruppe eine Methyl-, Ethyl-, Propyl-, Isopropyl-, n-Butyl-, tert.-Buthyl-, n-Pentyl- oder Isopentylgruppe vorliegen.

Weiterhin lassen sich Alkylidencyclohexene z.B. 4-Methylen- oder 4-Isopropylidencyclohex-1-en zu Alkylbenzolen umsetzen.

Besonders geeignet ist das erfindungsgemässe Verfahren für die Umsetzung von Cyclohexenen und/oder Cyclohexadienen, die an einem Kohlenstoffatom durch einen verzweigten oder unverzweigten Alkenylrest mit 2 bis 10, insbesondere 2 bis 8, vorzugsweise 2 bis 4 Kohlenstoffatomen substituiert sind und gegebenenfalls an einem anderen Kohlenstoffatom eine niedermolekulare Alkylgruppe mit 1 bis 4 Kohlenstoffatomen tragen, z.B. 4-Allyl-, 4-Pentenyl-, 1-Methyl-4-hexenyl-, 1-Isopropyl-4-vinyl-, 3-Vinyl- oder 2-Allyl-cyclohex-1-en, 5-Vinyl-cyclohexa-1, 3-dien oder 3-Isobutenyl-6-methyl-cyclohexa-1, 4-dien.

Insbesondere Alkenylcyclohexene der allgemeinen Formel (I)

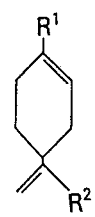

(I),

in der die Reste $R^1$ und $R^2$ unabhängig voneinander Wasserstoff oder eine niedermolekulare Alkylgruppe mit 1 bis 4 Kohlenstoffatomen bedeuten, z.B. 1-Methyl-4-vinyl- oder 4-Vinyl-cyclohex-1-en, lassen sich leicht zu Alkylbenzolen der Formel (II)

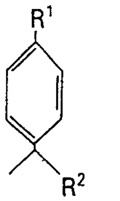

(II),

in der $R^1$ und $R^2$ die obengenannten Bedeutungen haben, umsetzen.

Nach dem erfindungsgemässen Verfahren lassen sich monocyclische ungesättigte Terpene, z.B. Sesquiterpene wie Bisabolen oder Zingiberen umsetzen. Besonders vorteilhaft ist die Umsetzung monocyclischer Monoterpene wie $\Delta^3$-Menthen, 3-Isopropyl-6-methylen-cyclohex-1-en, α-Terpinen und insbesondere α-Limonen zu p-Cymol. Anstelle von Limonen können auch die isomeren bicyclischen Monoterpene, bei denen das mittlere C-Atom der Isopropylgruppe mit einem zweiten C-Atom des Cyclohexenringes verbunden ist, verwendet werden, z.B. α-Pinen, β-Pinen oder $\Delta^3$-Caren. Zur Herstellung von p-Cymol kann auch vorteilhaft ein Gemisch der isomeren Terpene der Summenformel $C_{10}H_{16}$ verwendet werden oder Terpentinöl.

Die Überführung der Ausgangsstoffe in Alkylbenzole erfolgt in der Flüssigphase bei Temperaturen von 30 bis 300°C, vorzugsweise 50 bis 200°C oder vorteilhaft in der Gasphase bei Temperaturen von 100 bis 500, insbesondere 200 bis 400, vorzugsweise 250 bis 300°C. Die Belastung (WHSV) beträgt 0,1 bis 20, vorzugsweise 0,5 bis 5 g Ausgangsstoff pro Gramm Katalysator und Stunde.

Die Umsetzung kann in Gegenwart eines inerten Lösungsmittels durchgeführt werden, das in einer Menge von 20 bis 80 Gew.%, bezogen auf den Ausgangsstoff verwendet werden kann. In der Regel werden die Ausgangsstoffe jedoch direkt ohne Zusatz von Lösungsmitteln umgesetzt.

Als Katalysatoren für die erfindungsgemässe Reaktion werden acide zeolithische Katalysatoren des Pentasiltyps eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluss von Kationen in den Kristall, z.B. eines Alkali- oder Wasserstoffions, ausgeglichen. Ein Kationenaustausch ist möglich, die Räume zwischen den Tetraedern sind vor der Dehydratation durch Trocknen bzw. Calcinieren von Wassermolekeln besetzt. Die Zeolithe können auch anstelle des Aluminiums andere dreiwertige Elemente wie B, Ga, Fe oder Cr und anstelle des Siliciums andere vierwertige Elemente, wie Ge, enthalten.

Diese Zeolithe können unterschiedliche chemische Zusammensetzungen aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische. Besonders bevorzugt sind Alumino-, Boro- und Eisensilikatzeolithe des Pentasiltyps, sowie die in der DE-OS 3 006 471 beschriebenen isotaktischen Zeolithe.

Der Aluminosilikatzeolith kann z.B. aus einer Aluminiumverbindung, vorzugsweise $Al(OH)_3$ oder $Al_2(SO_4)_3$, und einer Siliciumkomponente, vorzugsweise hochdispersem Siliciumdioxid in wässriger Aminlösung, insbesondere in 1,6-Hexandiamin- oder 1,3-Propandiamin- oder Triethylentetramin-Lösung mit und ohne Alkali- oder Erdalkalizusatz bei 100 bis 220°C unter autogenem Druck hergestellt werden. Die so erhaltenen Aluminosilikatzeolithe enthalten je nach Wahl der Einsatzstoffmengen ein $SiO_2/Al_2O_3$-Verhältnis von 10 bis 40.000. Die Aluminosilikatzeolithe lassen sich auch in etherischem Medium, wie Diethylenglykoldimethylether, in alkoholischem Medium, wie Methanol bzw. 1,4-Butandiol oder in Wasser herstellen.

Der Borosilikatzeolith kann z.B. bei 90 bis 200°C unter autogenem Druck synthetisiert werden, indem man eine Borverbindung, z.B. $H_3BO_3$, mit einer Siliciumverbindung, vorzugsweise mit hochdispersem Siliciumdioxid, in wässriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Man kann bei dieser Reaktion anstelle einer wässrigen Aminlösung eine etherische Lösung, z.B. mit Diethylenglykoldimethylether, oder eine alkoholische Lösung, z.B. mit 1,6-Hexandiol als Lösungsmittel verwenden.

Den Eisensilikatzeolith erhält man z.B. aus einer Eisenverbindung, vorzugsweise $Fe_2(SO_4)_3$ und einer Siliciumverbindung, vorzugsweise auch hochdispersem Siliciumdioxid in wässriger Aminlösung, insbesondere 1,6-Hexandiamin, mit und ohne Alkali- oder Erdalkalizusatz bei 100 bis 200°C unter autogenem Druck.

Die so hergestellten Alumino-, Boro- und Eisensilikatzeolithe können nach ihrer Isolierung, Trocknung bei 100 bis 160°C, vorzugsweise 110°C, und Calcination bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge bei 110°C/16 h getrocknet und bei 500°C/16 h calciniert. Besonders vorteilhaft lassen sich solche Katalysatoren dadurch herstellen, dass man den isolierten Alumino- bzw. Boro- bzw. Eisensilikatzeolith direkt nach der Trocknung verformt und erstmals nach der Verformung einer Calcinierung unterwirft. Aus dem zu Strängen verformten Katalysator kann man durch Mahlen und Sieben Wirbelgut z.B. mit einer Teilchengrösse von 0,1 bis 0,6 mm erhalten. Die Alumino-, Boro- und Eisensilikatzeolithe können aber auch in reiner Form, ohne Binder, als Stränge oder Tabletten eingesetzt werden. Diese Verformung erfolgt unter Zusatz von Verstrangungs- oder Peptisierungshilfsmitteln, wie z.B. Hexaethylcellulose, Kartoffelstärke, Ameisensäure, Essigsäure, Oxalsäure, Salpetersäure, Ammoniak, Amin, Silikoester, Graphit oder deren Gemische.

Liegt der Zeolith aufgrund der Art seiner Herstellung nicht in der aciden H-Form, sondern in der Na-Form vor, dann kann diese durch Ionenaustausch mit Ammoniumionen und anschliessende Calcinierung oder durch Behandlung mit Säuren vollkommen oder partiell in die gewünschte H-Form übergeführt werden. Man kann an den Zeolithen zur Erhöhung der Selektivität, der Standzeit und der Anzahl der Regenerierungen auch unterschiedliche Modifizierungen vornehmen. Eine geeignete Modifizierung besteht z.B. darin, dass man den unverformten oder verformten Zeolithen mit Alkalimetallen wie Na – sofern nicht schon von der Synthese her die Alkali-Form des Zeolithen vorliegt – mit Erdalkali wie Ca, Mg und Erdmetallen wie B, Tl ionenaustauschen bzw. imprägniert. Insbesondere ist eine Dotierung der Zeolithe mit Übergangsmetallen wie Mo, W, Fe, Zn, Cu, Ni, mit Edelmetallen, wie Pd und Pt und mit seltenen Erdmetallen, wie Ce und La, vorteilhaft.

Praktisch stellt man solche modifizierten Kontakte z.B. so her, dass man den verformten Pentasilzeolithen in einem Steigrohr vorlegt und bei 20 bis 100°C z.B. eine wässrige oder ammoniakalische Lösung eines Halogenids oder eines Nitrats der voranbeschriebenen Metalle darüberleitet. Ein derartiger Ionenaustausch kann z.B. an der Wasserstoff-, Ammonium- und Alkaliform des Pentasil-Zeolithen vorgenommen werden. Man kann die Metallaufbringung auf den Zeolithen z.B. auch so vornehmen, dass man das zeolithische Material z.B. mit einem Halogenid, einem Nitrat oder einem Oxid der voranbeschriebenen Metalle in wässriger, alkoholischer oder ammoniakalischer Lösung imprägniert. Sowohl an einen Ionenaustausch als auch an eine Imprägnierung schliesst sich zumindest eine Trocknung und wahlweise eine abermalige Calcinierung an.

Im einzelnen verfährt man z.B. so, dass man Nickelnitrat $Ni(NO_3)_2 \cdot 6\,H_2O$ oder $Ce(NO_3)_3 \cdot 6H_2O$ in Wasser löst. Mit dieser Lösung wird dann der mit oder ohne Bindemittel verstrangte oder der unverstrangte Pentasilzeolith eine gewisse Zeit, ca. 30 min, getränkt. Die überstehende Lösung wird am Rotationsverdampfer von Wasser befreit. Danach wird der getränkte Zeolith bei ca. 150°C getrocknet und bei ca. 550°C calciniert. Dieser Tränkvorgang kann – wenn nötig – mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C von Calcination bei ca. 500°C kann das so gewonnene zeolithische Material mit und ohne Bindemittel zu Strängen oder Pellets oder Wirbelgut weiterverarbeitet werden.

Ein Ionenaustausch des in der H-Form vorliegenden Zeolithen kann so vorgenommen werden, dass man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht z.B. darin, dass man das zeolithische Material – verformt oder unverformt – einer Behandlung mit Säuren wie Salzsäure, Flussäure und Phosphorsäure und/oder mit Wasserdampf unterwirft. Dabei geht man vorteilhaft so vor, dass man das zeolithische Pulver vor seiner Verformung mit 0,001 n bis 2 n, bevorzugt 0,05 n bis 0,5 n Flussäure 1 bis 3 h unter Rückfluss behandelt. Nach Abfiltrieren und Auswaschen wird bei 100 bis 160°C getrocknet und bei 400 bis 550°C calciniert. Auch eine Behandlung der Zeolithe nach ihrer Verformung mit Bindemittel mit Salzsäure kann zweckmässig sein. Hierbei wird der Zeolith z.B. 1 bis 3 h zwischen 60 und 80°C mit einer 3 bis 25%igen, insbesondere mit einer 12 bis 20%igen Salzsäure behandelt, anschliessend ausgewaschen, bei 100 bis 160°C getrocknet und bei 400 bis 550°C calciniert. Man kann den Zeolithen auch durch Aufbringen von Phosphorverbindungen, wie Trimethoxyphosphat, modifizieren.

Nach einer eventuellen Desaktivierung der zeolithischen Katalysatoren, die beim Verfahren der Erfindung durch Koksabscheidung eintreten kann, lassen sich diese durch Abtrennen der Koksablagerung mit Luft oder mit einem Luft/$N_2$-Gemisch bei 400 bis 550°C, in einfacher Weise regenerieren, wodurch sie ihre Anfangsaktivität zurückerhalten. Man kann auch durch eine partielle Verkokung (pre-coke) die Aktivität des Katalysators für ein Selektivitätsoptimum des gewünschten Reaktionsproduktes einstellen. Wird die Reaktion in Gegenwart von Gasen wie Wasserstoff, Stickstoff und Wasserdampf ausgeführt, so kann damit die Produktzusammensetzung und Standzeit des Katalysators beeinflusst werden. Im allgemeinen werden die Katalysatoren wahlweise als 2 bis 4 mm Stränge, als Tabletten mit 3 bis 5 mm Durchmesser, als Pulver mit Teilchengrössen von 0,3 bis 0,5 mm oder als Wirbelkontakt von 0,1 bis 0,6 mm eingesetzt.

Das erfindungsgemässe Verfahren kann chargenweise oder kontinuierlich bei Normaldruck oder erhöhtem Druck nach den dafür üblichen Techniken, z.B. in einem Strömungsreaktor, Rührkessel oder Wirbelbettreaktor durchgeführt werden.

Nach der Umsetzung werden die entstandenen Alkylbenzole nach den dafür üblichen Techniken z.B. durch Destillation aus dem Reaktionsgemisch isoliert und nichtumgesetzte Ausgangsstoffe werden gegebenenfalls abgetrennt, um sie erneut für die erfindungsgemässe Umsetzung zu verwenden.

Nach dem erfindungsgemässen Verfahren lassen sich chemisch leicht zugängliche Verbindungen wie Vinylcyclohexen, das z.B. durch Dimerisierung von Butadien entsteht oder wie Limonen, α- und β-Pinen, die in vielen Pflanzen enthalten sind oder bei verschiedenen industriellen Prozessen in beträchtlicher Menge als Nebenprodukte anfallen, in wichtige Zwischenprodukte überführen. P-Cymol dient beispielsweise für die Herstellung von p-Kresol. Durch Dehydrierung von Ethylbenzol ist Styrol zugänglich, das z.B. für die Synthese von Polystyrol dient.

Beispiele 1 bis 12

Die Reaktion wurde wie folgt durchgeführt:

Limonen, α-, β-Pinen und Vinylcyclohexen wurden unter isothermen Bedingungen in einem Rohrreaktor eingesetzt und in der Gasphase bei Temperaturen zwischen 200 und 300°C über einen Zeolithkatalysator geleitet. Die erhaltenen Reaktionsprodukte wurden destillativ aufgearbeitet und durch Siedepunkt, Brechungsindex und NMR-Spektren charakterisiert.

Die quantitative Bestimmung der Reaktionsprodukte und der Ausgangsprodukte erfolgte gaschromatographisch. Die Art des Katalysators, die Temperatur, die Belastung (WHSV), der Umsatz und die Selektivität sind den folgenden Tabellen zu entnehmen.

Als Katalysatoren wurden eingesetzt:

Katalysator A

Der Borosilikatzeolith wurde in einer hydrothermalen Synthese aus 640 g $SiO_2$ (hochdisperse Kieselsäure), 122 g $H_3BO_3$, 8000 g einer wässrigen 1,6-Hexandiamin-Lösung (Mischung 50:50 Gew.%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/24 h calciniert. Es wurde ein Borosilikatzeolith vom Pentasiltyp erhalten, der 94,2 Gew.% $SiO_2$ und 2,3 Gew.% $B_2O_3$ enthielt.

Der Borosilikatzeolith wurde mit Boehmit im Gewichtsverhältnis 60:40 zu 2 mm Strängen verformt, bei 110°/16 h getrocknet und bei 500°C/24 h calciniert.

Katalysator B

Die Stränge von Katalysator A wurden mit ammoniakalischer $Pd(NO_3)_2$-Lösung behandelt. Der Pd-Gehalt des Katalysators beträgt 3,3 Gew.%.

Katalysator C

Der unter Katalysator A beschriebene verformte Bor-Zeolith wird mit $Ni(NO_3)_2 \cdot 6\,H_2O$ imprägniert. Der Ni-Gehalt des fertigen Katalysators beträgt 3,5 Gew.%.

Katalysator D

Der Borosilikatzeolith wie bei Katalysator A beschrieben wurde ohne Bindemittel zu 2 mm-Strängen verformt, bei 110°C/16 h getrocknet und bei 500°C/16 h calciniert. 100 g dieser Stränge wurden mit einer Pd und Ce enthaltenden Lösung getränkt. Die eingesetzte Lösung setzt sich zusammen aus 15,5 g einer 11%igen ammoniakalischen $Pd(NO_3)_2$-Lösung und 13,3 g $Ce(NO_3)_3 \cdot 6\,H_2O$ in 80 g $H_2O$. Nach Befreien von überstehendem Restwasser wird bei 130°C/2 h getrocknet und bei 540°C/2 h calciniert. Der Pd-Gehalt des Katalysators beträgt 1,5 Gew.%, der Ce-Gehalt 3,5 Gew.%.

Katalysator E

Ein Aluminosilikatzeolith vom Pentasiltyp wurde unter hydrothermalen Bedingungen bei autogenem Druck und 150°C aus 650 g hochdispersem $SiO_2$, 203 g $Al_2(SO_4)_3 \cdot 18\,H_2O$ in 10 kg einer wässrigen 1,6-Hexandiamin-Lösung (Mischung 50 : 50 Gew.%) in einem Rührautoklaven hergestellt. Nach Abfiltrieren und Auswaschen wurde das kristalline Reaktionsprodukt bei 110°C/24 h getrocknet und bei 500°C/24 h calciniert. Dieser Aluminosilikatzeolith enthielt 91,6 Gew.% $SiO_2$ und 4,6 Gew.% $Al_2O_3$.

Aus diesem Zeolith wurden durch Verformen 2 mm-Stränge hergestellt, die bei 100°C/16 h getrocknet und bei 500°C/16 h calciniert wurden.

Katalysator F

Katalysator F wird durch Imprägnierung von Katalysator E mit einer wässrigen $PD(NO_3)_2$-Lösung (11%ig) erhalten. Der Pd-Gehalt des bei 540°C calcinierten Katalysators beträgt 3,2 Gew.%.

Katalysator G

Katalysator G wird erhalten wie Katalysator D, nur wird der Aluminosilikatzeolith wie bei Katalysator E beschrieben eingesetzt. Der Pd-Gehalt beträgt 1,5 Gew.%, der Ce-Gehalt 3,6 Gew.%.

Tabelle 1

Umsetzung von α-Limonen, α- und β-Pinen

| Beispiel | Einsatzstoff | Katalysator | Temperatur °C | WHSV $h^{-1}$ | Umsatz % | Selektivität p-Cymol | [a] in % MIC[b] |
|---|---|---|---|---|---|---|---|
| 1 | α-Limonen | A | 200 | 2,4 | 100 | 20,8 | 16,1 |
| 2 | α-Limonen | B | 200 | 2,4 | 100 | 70,0 | 13,0 |
| 3 | α-Limonen | C | 200 | 2,4 | 100 | 47,5 | 4,2 |
| 4 | α-Limonen | D | 150 | 1,7 | 100 | 75,2 | 24,4 |

Umsetzung von α-Limonen, α- und β-Pinen

| Beispiel | Einsatzstoff | Katalysator | Temperatur °C | WHSV $h^{-1}$ | Umsatz % | Selektivität p-Cymol | [a] in % MIC[b] |
|---|---|---|---|---|---|---|---|
| 5 | α-Limonen | D | 200 | 1,7 | 100 | 86,6 | 11,4 |
| 6 | α-Limonen | E | 200 | 2,4 | 100 | 44,5 | 12,8 |
| 7 | α-Limonen | F | 200 | 2,4 | 100 | 73,5 | 11,0 |
| 8 | α-Limonen | G | 200 | 2,4 | 100 | 73,7 | 11,7 |
| 9 | α-Pinen | B | 200 | 2,0 | 96,8 | 57,0 | 11,4 |
| 10 | β-Pinen | B | 200 | 2,4 | 99,0 | 55,1 | 14,3 |

[a] bezogen auf umgesetzten Einsatzstoff
[b] MIC = Methylisopropylcyclohexan/Methylisopropylcyclohexen

Als weitere Nebenprodukte können Toluol und Xylol anfallen.

Tabelle 2

Umsetzung von Vinylcyclohexen

| Beispiel | Katalysator | Temperatur | WHSV | Umsatz | Selektivität in % [a] | |
|---|---|---|---|---|---|---|
| | | °C | $h^{-1}$ | % | Ethylbenzol | Ethylcyclohexan/ Ethylcyclohexen |
| 11 | B | 200 | 2,4 | 100 | 70,7 | 9,2 |
| 12 | D | 200 | 1,0 | 100 | 82,8 | — |

[a] bezogen auf umgesetztes Vinylcyclohexen

**Patentansprüche**

1. Verfahren zur Herstellung von Alkylbenzolen durch Umsetzung von Alkyl-, Alkyliden- und/oder Alkenylcyclohexenen sowie Alkyl- oder Alkenylcyclohexadienen in Gegenwart eines Katalysators, dadurch gekennzeichnet, dass man acide zeolithische Katalysatoren des Pentasiltyps in der Gas- oder Flüssigphase verwendet.

2. Verfahren nach Anspruch 1, dadurch gekennzeichnet, dass man Cyclohexene und/oder Cyclohexadiene umsetzt, die an einem Kohlenstoffatom durch eine Alkenylgruppe mit 2 bis 10 Kohlenstoffatomen substituiert sind und gegebenenfalls an einem anderen Kohlenstoffatom eine Alkylgruppe mit 1 bis 4 Kohlenstoffatomen tragen.

3. Verfahren nach Anspruch 1, dadurch gekennzeichnet, dass man Alkenylcyclohexene der Formel (I)

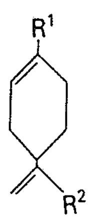

(I),

in der die Reste $R^1$ und $R^2$ unabhängig voneinander Wasserstoff oder eine niedermolekulare Alkylgruppe bedeuten, umsetzt.

4. Verfahren nach Anspruch 1, dadurch gekennzeichnet, dass man mono- oder bicyclische Monoterpene der Summenformel $C_{10}H_{16}$ wie α-Limonen, α- oder β-Pinen allein oder als Isomerengemisch umsetzt.

5. Verfahren nach Anspruch 1, dadurch gekennzeichnet, dass man die Umsetzung in der Flüssigphase bei Temperaturen von 30 bis 300°C oder in der Gasphase bei Temperaturen von 100 bis 500°C durchführt.

6. Verfahren nach Anspruch 1, dadurch gekennzeichnet, dass man als Katalysatoren Borosilikatzeolithe des Pentasiltyps einsetzt.

7. Verfahren nach Anspruch 1, dadurch gekennzeichnet, dass man als Katalysatoren Aluminiumsilikatzeolithe des Pentasiltyps einsetzt.

8. Verfahren nach Anspruch 1, dadurch gekennzeichnet, dass man als Katalysatoren Eisensilikatzeolithe des Pentasiltyps einsetzt.

9. Verfahren nach Anspruch 1, dadurch gekennzeichnet, dass man als Katalysatoren Übergangsmetall, Edelmetall und/oder seltene Erdmetall dotierte Pentasilzeolithe verwendet.

10. Verfahren nach Anspruch 9, dadurch gekennzeichnet, dass man als Dotierungsmetalle Ni, Pd, Ce oder deren Gemische verwendet.

**Revendications**

1. Procédé de préparation d'alkylbenzènes par réaction, d'alkyl-, alkyliden- et/ou alcénylcyclohexenes, ainsi qu'alkyl- ou alcényl-cyclohexadiène, en présence d'un catalyseur, caractérisé par le fait que l'on utilise dans la phase gazeuse ou liquide des catalyseurs zéolithiques acides du type pentasil.

2. Procédé selon la revendication 1, caractérisé par le fait que l'on fait réagir des cyclohexènes et/ou cyclohexadiènes qui sont substitués, sur un atome de carbone, par un groupe alcényle ayant 2 à 10 atomes de carbone et portent éventuellement, sur un autre atome de carbone, un groupe alkyle de 1 à 4 atomes de carbone.

3. Procédé selon la revendication 1, caractérisé par le fait que l'on fait réagir des alcénylcyclohexènes de formule I

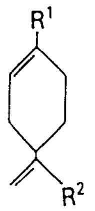

(I),

dans laquelle les restes $R^1$ et $R^2$ signifient, indépendamment l'un de l'autre, hydrogène ou un groupe alkyle de bas poids moléculaire.

4. Procédé selon la revendication 1, caractérisé par le fait que l'on fait réagir des monoterpenes mono- ou bicycliques de formule brute $C_{10}H_{16}$, tels que des α-limonène, α- ou β-pinène, seuls ou en mélange d'isomères.

5. Procédé selon la revendication 1, caractérisé par le fait que l'on effectue la réaction, dans la phase liquide à des températures de 30 à 300°C ou dans la phase gazeuse à des températures de 100 à 500°C.

6. Procédé selon la revendication 1, caractérisé par le fait que l'on introduit comme catalyseurs des zéolithes de borosilicate du type pentasil.

7. Procédé selon la revendication 1, caractérisé par le fait que l'on introduit comme catalyseurs des zéolithes de silicate d'aluminium du type pentasil.

8. Procédé selon la revendication 1, caractérisé par le fait que l'on introduit comme catalyseurs des zéolithes de silicate de fer du type pentasil.

9. Procédé selon la revendication 1, caractérisé par le fait que l'on utilise comme catalyseurs des pentasilzéolithes dopés par un métal de transition, un métal noble et/ou un métal des terres rares.

10. Procédé selon la revendication 9, caractérisé par le fait que l'on utilise, comme métaux de dopage, Ni, Pd, Ce ou leurs mélanges.

**Claims**

1. A process for the preparation of an alkylbenzene by converting an alkyl, an alkylidenecyclohexene and/or an alkenylcyclohexene or an alkyl- or an alkenylcyclohexadiene in the presence of a catalyst, wherein an acidic zeolite catalyst of the pentasil type is used and the procedure is carried out in the gas phase or liquid phase.

2. A process as claimed in claim 1, wherein cyclohexenes and/or cyclohexadienes which are substituted at a carbon atom by alkenyl of 2 to 10 carbon atoms and may carry an alkyl group of 1 to 4 carbon atoms on another carbon atom are converted.

3. A process as claimed in claim 1, wherein an alkenylcyclohexene of the formula (I)

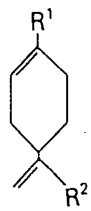

(I),

where $R^1$ and $R^2$ independently of one another are each hydrogen or a low molecular weight alkyl group, is converted.

4. A process as claimed in claim 1, wherein a mono-or bicyclic monoterpene of the empirical formula $C_{10}H_{16}$, such as α-limonene or α- or β-pinene, is converted alone or in the form of an isomer mixture.

3. A process as claimed in claim 1, wherein the reaction is carried out in the liquid phase at from 30 to 300°C or in the gas phase at from 100 to 500°C.

6. A process as claimed in claim 1, wherein the catalyst used is a borosilicate zeolite of the pentasil type.

7. A process as claimed in claim 1, wherein the catalyst used is an aluminosilicate zeolite of the pentasil type.

8. A process as claimed in claim 1, wherein the catalyst used is an iron silicate zeolite of the pentasil type.

9. A process as claimed in claim 1, wherein the catalyst used is a pentasil zeolite doped with a transition metal, a noble metal and/or a rare earth metal.

10. A process as claimed in claim 9, wherein Ni, Pd, Ce or a mixture of these is used as a dopant.